(19) Europäisches Patentamt
European Patent Office
Office européen des brevets

(11) **EP 4 151 276 A1**

(12) **EUROPEAN PATENT APPLICATION**

(43) Date of publication:
**22.03.2023   Bulletin 2023/12**

(21) Application number: **21197632.9**

(22) Date of filing: **20.09.2021**

(51) International Patent Classification (IPC):
**A61N 5/10** (2006.01)        **A61B 5/00** (2006.01)
**A61B 5/01** (2006.01)        **A61B 6/00** (2006.01)
**G06T 7/00** (2006.01)

(52) Cooperative Patent Classification (CPC):
**A61N 5/1048; A61B 5/445; A61B 5/7264;**
**A61B 6/4417; A61B 6/542; A61N 5/0614;**
**A61N 5/1071; G06T 7/0012;** A61B 5/0077;
A61B 5/015; A61B 5/4842; A61B 6/032;
A61B 6/461; A61B 2505/05; A61N 2005/0628;

(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **VIK, Torbjoern**
  **Eindhoven (NL)**

• **HEESE, Harald Sepp**
  **Eindhoven (NL)**
• **WEESE, Rolf Jürgen**
  **Eindhoven (NL)**
• **ISOLA, Alfonso Agatino**
  **Eindhoven (NL)**

(74) Representative: **Philips Intellectual Property &
Standards
High Tech Campus 52
5656 AG Eindhoven (NL)**

(54) **MONITORING AND DETECTION OF CUTANEOUS REACTIONS CAUSED BY RADIOTHERAPY**

(57)    A system (SYS) and related method for monitoring exposure to radiation, preferably as received during radiation therapy. The system comprises an input interface (IN) or receiving input data including sensed data of a portion of a subject's skin acquired a sensor (SN) over time. A predictor logic (PL) processes the input data to compute output data including a map-The map is representative of spatially resolved magnitudes and/or type of predicted skin reactions to radiation exposure. The system may be used to predict adverse skin reactions as a result of radiation therapy, or due to skin exposure to other types of radiation.

FIG. 2

**(Cont. next page)**

(52)  Cooperative Patent Classification (CPC): (Cont.)
      A61N 2005/1059; A61N 2005/1074;
      G06T 2207/10016; G06T 2207/20084;
      G06T 2207/30088

**Description**

FIELD OF THE INVENTION

The invention relates to a system for monitoring exposure to radiation, to a system for training a machine learning model for use in such a system, to a system of generating training data for training a machine learning model, to related methods, to a computer program element and to a computer readable medium.

BACKGROUND OF THE INVENTION

Cancer is a leading cause of death in particular in industrialized nations around the world. Well over half of cancer patients receive treatment by radiotherapy ("RT"). Types of RT includes external RT, where a high energy ionizing radiation is applied to the patient from the outside, and internal RT, such as brachytherapy. The goal is to destroy cancerous tissue whilst preserving non-cancerous, healthy tissue. In some types of external RT, X-radiation in the megavolt (MV) range is used. The total radiation dose is administered in portions (also known as "fractions") over time as per a treatment plan, such as once per day over the course of 2-4 weeks for example.

In external radiotherapy, treatment plans (which may prescribe among other parameters collimator apertures and control point weights etc) are optimized, to ensure that a prescribed dose to a target region is deposited there, whilst dose exposure of healthy tissue outside the target is minimized. Typically, different trade-offs between risk organs and target regions are possible, each having different probability of toxic side effects. In some clinical settings, plans may be adapted during the treatment (typically comprising 25-30 fractions) based on anatomical changes for example, that occur as a result of treatment.

About 95% of patients who receive RT suffer from either acute or chronic skin injuries as a result from RT. Severity of toxicity may depend on external factors (treatment plan, beam type, ...) and intrinsic factors (genes, gender, obesity, and others). It can have significant impact on quality of life for the patient.

Skin injury can be reported by patients themselves (for example, via Patient Reported Outcome Measurements - PROMs) or by clinicians, for example, at regular clinical control visits. Side effects can appear instantly or delayed, even up to several months after RT treatment. The type of medical attention that such skin injury commands may depend on severity, and could range from application of skin lotion to treatment interruption.

RT patients might not notice any side effects, such as inflammatory effects or other, during a single treatment session, but only after several fractions, and possible with a time lag after delivery.

As mentioned, some such skin injury may cause treatment abort, which can have devastating health consequences as the caner may proliferate, may squander time resources, and may put strain on the treatment equipment to the detriment of other patients.

SUMMARY OF THE INVENTION

There may therefore be a need for a more efficient use of RT procedure and/or equipment. In particular, there may be a need in reducing side-effects in RT.

An objective of the present invention is achieved by the subject matter of the independent claims where further embodiments are incorporated in the dependent claims. It should be noted that the following described aspect of the invention equally applies to the system for training a machine learning model, to the system of generating training data for training the machine learning model, to the related methods, to the computer program element and to the computer readable medium.

According to a first aspect of the invention there is provided a system for monitoring exposure to radiation, comprising:

input interface or receiving input data including sensed data of a portion of a subject's skin acquired by at least one sensor over time,

A predictor logic configured to process the said input data to compute output data including a map representative of spatially resolved magnitudes and/or type of predicted skin reactions to radiation exposure; and

an output interface to output said map.

The system may be used to predict adverse skin reactions as a result of radiation therapy, or due to skin exposure to other types of radiation. The sensed data is time series data of imagery, for example video feed acquired of at least a portion of subject's skin. Using such time series data has been found to make the predictions accurate and robust.

In embodiments, the system comprises a graphics display generator configured to cause displaying, on a display device, a graphics display of the map.

In embodiments, the map is color and/or grey-value coded, the values modulated with the skin reaction magnitudes or type as computed.

In embodiments, the system comprises a planning module (PM) configured to compute a treatment plan for the subject based at least on the map.

In embodiments, the, the one or more sensors include any one or more of: i) optical camera, ii) depth or range sensing camera, iii) IR or near-IF camera.

The depth or range sensing camera such as LIDAR or other could be useful harnessed herein to create a surface map of the body. This may be registered (spatially aligned) with a planning image such as a 3D image (eg, pre-sim-CT model) of the patient/subject.

In embodiments, the input data further includes contextual data such as one or more control parameter(s) for a radiation treatment delivery apparatus and/or treatment delivery parameter(s). Alternatively or in addition, the input data includes vital sign measurements in relation to the subject, including any one or more of i) skin temperature, ii) oxygenation, iii) pule rate, iv) blood pressure. The vital sign measurement are other examples of contextual data. More generally, the contextual data may describe attributes or the subject exposed to radiation, or contextual data may describe the manner in which the radiation is generated and/or the manner of exposure. using contextual data preferably in combination with the sensed data, makes the prediction more robust. Like the sensed data, the contextual data, in particular the vital sign measurements may also be times series data.

The radiation delivery parameters may be grabbed from a logfile of the actually delivered beam generated by an RT delivery system, such as collimator leaf positions.

In embodiments, the predictor logic is based on a machine learning model.

In embodiments, the machine learning model includes am artificial neural network. A convolutional network may be used for example, but other ML models not necessary of the artificial neural network type are also envisaged herein.

In embodiments, the sensed data is acquired during radiation exposure, such as during a fraction deliver in radiation therapy.

In embodiments, the said radiation exposure is one during any one of: i) radiation therapy, ii) X-ray imaging, iii) sunbathing, iv) UV-sunbed use.

Other uses than medical (RT or imaging with X-ray) are also envisaged. The system may be at least partly implemented as an application run a mobile end user device (smartphone, tablet, etc) during recreational radiation use for example.

In another aspect there is provided a computer system for training the machine learning model based on training data.

The above-mentioned sensor setup, or other sensors, may be used in a data collection phase prior to training the model. The sensor(s) may thus be part of a training data generator system for procuring or generating the training data for use in training the machine learning model. The system may include the sensor(s) and a processing system including a memory to store the training data as acquired. The acquired over time data (such as imagery video) form training data input data. This may be annotated by a human export or automatically to obtain associated target data.

What is proposed herein is to monitor patient's skin surface during treatment delivery with one or more cameras for example to acquire time series of imagery. Optionally, vital signs, temperature and / or oxygenation are measured and recorded as time series of contextual data. The time series of imagery, optionally in combination with the time series of contextual data, may be used by a machine-learning algorithm/model to predict type and severity of skin injury. The output produced by the ML algorithm/model may include a skin map that represents per on-skin position or per on-skin region, severity and/or type of skin lesion caused by treatment radiation exposure.

The predicted skin injury may be resolved for magnitude and/or type of skin changes/reactions. This is beneficial as different types of skin reaction may call for different types of treatment. For example, the said type may include any one of swelling, skin redness, pigmentation, fibrosis, ulceration, pain, skin warm to touch, burning, and itches, etc.

In another aspect there is provided a computer-implemented method of monitoring exposure to radiation, comprising :

receiving input data including sensed data of a portion of a subject's skin acquired by at least one sensor over time, processing the said input data to compute output data including a map representative of spatially resolved magnitudes and/or type of predicted skin reactions to radiation exposure; and outputting said map.
preferably, machine learning is used in the processing of the input data.

In another aspect there is provide a method for training the machine learning model, based in training data.

In another aspect, there is provided a method for procuring or generating training data for use in training the machine learning model of claim 8 or 9, based on training data.

In another aspect, there is provided a computer program element, which, when being executed by at least one processing unit, is adapted to cause the processing unit to perform any one of the methods.

In another aspect, there is provided at least one computer readable medium having stored thereon the program element, or having stored thereon the trained machine learning model.

*"user"* relates to a person, such as medical personnel or other, operating the imaging/treatment apparatus or overseeing the imaging/treatment procedure. In other words, the user is in general not the subject in clinical setting. *"Subject"* as used herein includes a human or animal patient (eg, pets or husbandry) who/that is treated, but may also relate to the leisure user who seeks out radiation exposure for pleasure for example. In a clinical setup, the *"subject"* may also be referred to herein as *"[the] patient"*.

BRIEF DESCRIPTION OF THE DRAWINGS

Exemplary embodiments of the invention will now be described with reference to the following drawings, which, unless stated otherwise, are not to scale, wherein:

Fig. 1 shows a block diagram of a schematic diagram of an arrangement for radiation therapy;
Fig. 2 shows a block diagram of a monitoring system that may be used in the arrangement of Fig. 1 according to one embodiment;
Fig. 3 shows a block diagram of a machine learning model as may be used in the system of Figure 2 in embodiments;
Fig. 4 shows a block diagram of a computer implemented training system for training a machine learning model based on training data;
Fig. 5 shows a flow chart of a computer-implemented method of supporting a radiation therapy session; and
Fig. 6 shows a flow chart of a method of training a machine learning model based on training data.

DETAILED DESCRIPTION OF EMBODIMENTS

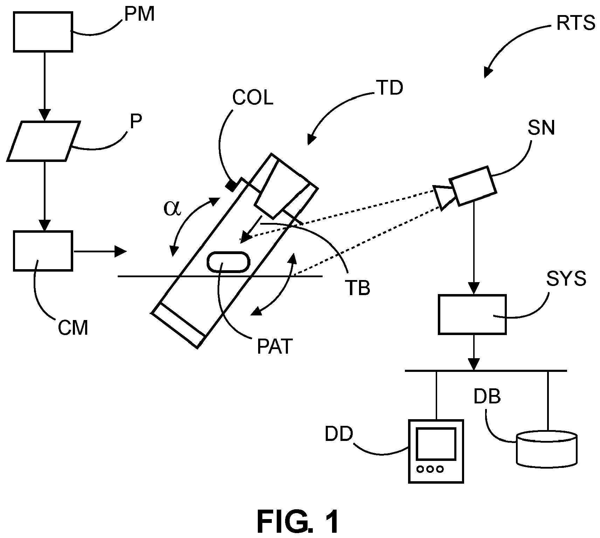

Reference is now made to Fig. 1 which shows a schematic block diagram of a radiation therapy system RTS.
The system RTS may include a treatment device TD such as a linear accelerator ("linac"). The treatment device TD includes a treatment head for generating a high energy radiation beam TB for external beam therapy. At least a part of a patient, a target volume ("TV") of a region of interest ROI, is irradiated by exposure to the treatment beam TB. The target volume may include tumor tissue to be treated.
The treatment head is preferably arranged on a rotatable gantry, rotatable around the patient so that the target volume is exposed from different treatment angles $\alpha$ along which the beam is made to propagated through the TV. In addition or instead of exposure from different angles, the treatment head my include a collimator COL operable to modulate treatment beam TB's cross sectional shape to conform along substantially each propagation direction to a physical shape of the target volume TV.
Operation of the treatment device TD is controlled from a control module CM. The control module CM processes a treatment plan P. The treatment plan P may include a control sequence according to which various operational functions of the delivery device TD is controlled, including any one or more of: the adjustment of the beam cross section in shape and size, the (propagation) gantry angle $\alpha$, beam energy, exposure time, and so forth. During treatment, a certain radiation dose is deposited in the target volume. The control program is configured so that a sufficient amount of radiation dose is deposited at the target volume to kill off tumor cells, thus stunning proliferation of cancer cells. At the same time, the program is configured to ensure that organs at risk ("OAR") (healthy, non-tumorous tissue) situated around the target volume TV are not to receive more than a certain maximum dosage. The cross sectional shape and changeover between treatment angles $\alpha$ are adjusted and controlled so that the treatment beam delivered along the different directions $\alpha$ intersect at the target volume so as to deliver a higher dose there, and thus keeping dosage at OAR within defined dosage constraints.
The machine control sequence (and/or other plan components such as scheduling of and amount of fraction, etc) is computed by a planning module PM. The planning module PM may be implemented as a computing system that runs a planning algorithm. The desired control parameters may be derived as solution to an optimization problem. The optimization problem may be formulated into terms of an objective function such as a cost function. The cost function is based on a set of dose constraints. The dose constraints are set by a dosimetrist or other medical professional. The dose constraints may be prescribed or defined on an initial planning image of the patient that represents the region of interest including the TV. The TV may be defined by segmentation or other. Annotations thereof may include the above-mentioned dose constraints that prescribe the maximum dosage allowed at organ(s) at risk and/or the minimum dose that needs to be delivered at the TV.
In more detail, the planning image is acquired prior to planning by a suitable imaging modality, usually CT (computed tomography), sometimes MRI (magnetic resonance imaging). The target volume TV and OARs may be defined in the planning image of the ROI by manual, semi-automatic or automatic segmentation for example. Dose constraints in relation to TV and OAR may be spatially defined based on the planning image. An iterative numerical optimization procedure may be applied to improve the cost function to obtain the control parameters. Once an optimal solution (the said parameter) is found, the control parameters (including machine settings, etc) may be made available as treatment plan P which may then be used to commence treatment in one or more session to control the treatment delivery device TD. The overall dose may be delivered in parts ("fractions") over a course of one or more time intervals (usually daily, over one or more weeks for example) in multiple treatment sessions, one fraction per session, until, at the conclusion of the last fraction, the whole dosage as per plan P was delivered. In stereotactic treatment, only a single (or a few) fraction(s) is/are delivered. The plan P may be changed based on physiological changes that may occur during treatment delivery.

As mentioned earlier, a number of patients will present with adverse skin reactions due to the treatment radiation beam TB exposure. Various degrees of toxicity have been reported. Acute or chronic skin injuries may be the result of the radiation therapy as an undesirable side effect. Such skin injuries are caused by the high energy radiation beam penetrating the skin and at least partly damaging skin cells. The skin injuries may not be noticed by the patient during a single treatment session, but may only become apparent after several fractions were delivered, possible delayed with a time lag post-delivery.

The relationship between the control parameters (such as beam energy, aperture shape, dose rate, treatment direction angle $\alpha$, etc) and acute observable skin reactions (such as oxygenation, local temperature, etc) are not fully understood. What is also not understood is how the observable skin reactions may translate into localized skin conditions/injury/disease at respective points on the patient's skin.

It is proposed herein to use in particular machine learning ("ML") to understand the above described relationships, on the one hand between control/treatment parameters and acute skin reactions, and the development of skin disease at a later stage on the other hand. It appears nay impossible to model these relationships analytically. However, machine learning opens up an option where no such explicit analytical modelling is required, but where approximations of the above mentioned latent relationships between treatment parameters versus skin reactions and skin reactions versus localized skin injuries or disease can be learned from a pool of prior training data. The training data may medical records, imagery, control parameters of historic treatment sessions of the same or other patients, as may be held in medial databases, such as in a HIS (hospital information system) or a PACS (picture archiving and communication system) or other such data repositories.

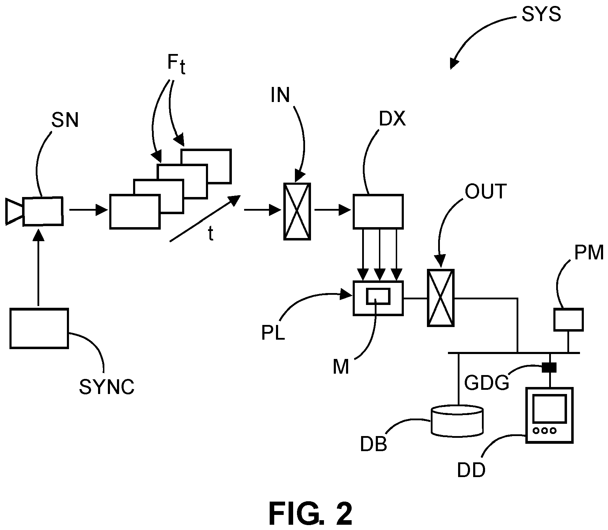

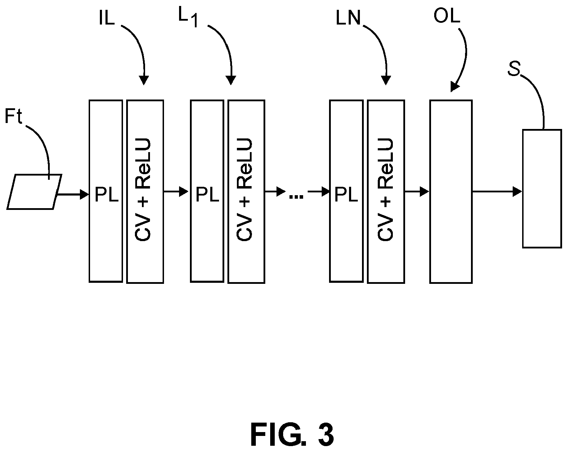

The radiation therapy system RTS may include the a novel monitoring (sub-)system SYS. The monitoring system SYS is shown schematically on the right hand side of Fig. 1, with more details in Fig. 2. The system may include a predictor logic PL. The logic PL may be based on a trained machine learning model M. A machine learning model M according to one embodiment, and its training based on training data will be explored more fully below at Figs. 3,4,6. For now, it will be assumed the model M was sufficiently trained in (one or more) prior training phases, and is ready for use in day-to-day clinical practice, a post-training phase referred to herein as deployment phase.

Input data for data processing by ML model M during deployment phase is supplied by one or more sensors SN. Reference will be made herein frequently to *"[the] sensor SN"*, with the understanding there may be more than one such sensor.

The sensor SN may be configured to monitor exposed skin region(s) of the patient during one or more (in particular all) radiation therapy sessions. The sensor may be a camera or video camera system. The sensors may be of different types providing different channels of information such as infra-red (IR), near-infra-red (NIR), or optical imagery or other still. In addition, contextual data, including vital sign data may be extracted from the acquired footage or imagery or may be measured separately by dedicated measurement probes, such as oximeter, thermometer, blood pressure measurement device, etc.

The contextual data may include the treatment parameters as may be collected from log files. The control parameters may include recording of treatment delivery parameters over time, such as any one or more of dose rate, beam angle, collimator aperture, etc. In addition or instead, the contextual data may control parameters as used to control operation of the treatment device TD. In addition still, the contextual data may include a patient surface model obtained from the planning CT image for example.

Inclusion of any or more (eg, all) of the above mentioned contextual data tends to make the ML data processing more robust. The contextual data may be used for displaying results of the analysis by model M. For example, using the control parameters and/or planning imagery as contextual data, allows training the model so that the resulting skin map is correctly associated with the relevant skin portion (location). The control parameters may allow the model to learn distinguishing a light vs severe skin reaction. For example, two patients receiving a similar momentum dose (beam intensity and angle) may present with different onset of reddish color, eg quick vs slow showing of side-effects, etc.

Preferably, the sensor SN is configured to acquire a stream of image data, such as a time series of imagery (a video feed), and the model M is configured to process such an image data stream, at once or suitably sized in one or more blocks, or frame-by- frame as received. As said, more than one such sensor SN for acquiring respective image data streams may be used. The sensors SN may be arranged at different locations in the treatment room. To improve the quality of the prediction, it might be necessary to remove body hair from the relevant skin surface prior to data acquisition by sensor SN.

The data streams may be jointly processed, or may be processed separately by model M or by different such models Mj (not shown). The vital sign measurements, if any, may be co-processed with the image steam data supplied by sensor(s) SN. Additional contextual data such as bio-characteristics of the patient (for example weight, sex, height etc) and/or their medical history may be so co-processed with image stream, and possibly with the vital sign measurements, if applicable and so desired.

The predictor logic PL of system SYS processes the input data (including in particular the video feed acquired by sensor SN) to produce/predict an indication on where, that is, at which on-skin position of the patient's skin, injury or skin disease may develop as a result of radiation therapy exposure. The type of injury may be predicted in addition or instead in similar, spatially resolved manner.

Operation of the monitoring system SYS is now explained in more detail with reference to Fig. 2. Fig. 2 shows a schematic block diagram of the monitoring system SYS which may be implemented on one or more computing systems.

Sensor SN operates during the treatment session to acquire the stream of data such as image frames Ft in the form of the video feed. Acquisition of the video feed may be gated or synchronized by a synchronizer SYNC in dependence on certain events at the treatment device TD. Frames of video feed $F_t$ are recorded at a suitable frame rate such as approximately *20-60 fps* or other. The feed Ft is received at an input port IN of system SYS. An optional data extractor DX may operate to extract vital sign measurements, such as oxygenation or temperature measurements, from the video stream, based on image processing techniques as reported elsewhere. Suitable fluctuations in color, hue, intensity etc, in the recorded feed, not necessarily discernable by the unaided eye, are analyzed by an image processor of data extractor DX to obtain the measurements. A suitable such camera-setup envisaged herein in embodiments was described by M Paul, et al in "A camera-based multispectral setup for remote vital signs assessment", published in H Eskola et al (Eds.), EMBEC & NBC 2017, IFMBE Proceedings, vol 65, Springer, Singapore, pp. 968-971 (2018). Such or similar camera system SN may be configured to measure perfusion levels that manifest in subtle color changes of the skin. IR or NIR cameras may be able to pick up skin temperature readings.

Alternatively, other measurement channels such as oximeters or thermometer measurement, blood pressure, etc, are used to procure contextual data as in-vivo measurements, rather than as extractions from the video feeds.

The input data such as the video feed, and optionally contextual data, capture even subtle skin reactions during exposure to the radiation beam.

The video feed (that is the time sequence of individual frames/images) and/or contextual data is acquired over a certain length of time. The length of time may cover the whole session, start / end, before / after the session, or a single or more disjoint parts thereof. Video feeds from multiple sessions may be co-processed by model M for more robustness and to recognize longer term trends. In other words, the model M of PL may be fed with the video footage or a given ongoing treatment session. Preferably, however, the current video feed Ft is concatenated with the one or more previous video feeds acquired during previous treatment sessions and it is the whole or parts of the video feeds acquired up to and including the current feed that are processed by module M.

The trained model M of predictor logic PL processes the input data including the feed $F_t$ of a certain length as mentioned, and outputs output data at output port OUT. The output data may be stored in a database for later reference or may be processed by a graphics display generator GDG into a graphics display GD which may be displayed on a monitor or display device DD. The graphics display GD may be part of a GUI. The graphics display GD may be part of the treatment planning system PM for example. Display of the graphics display GD may be effected during an ongoing treatment session, or anytime thereafter as required.

Alternatively, the input data comprises the time series of data extracted by extractor DX, instead of the video feed Ft, although this is less preferred. What is preferred herein is that the input (Ft, ct) comprises the video feed and the contextual data. However the contextual data is optional.

It has been found that the changing skin reactions recorded over the time in one or more such video feeds "encode" indicators as to whether or not a skin condition will develop at a certain point after treatment on the patient's skin exposed to the radiation treatment beam. Skin, on a microscopic level, presents with a jagged surface, comprising crests and troughs. This results in certain portions or points of the skin to be exposed differently to the incoming treatment radiation beam, thus eliciting perhaps different degrees and types of skin reactions. This skin constellation may lead to unfavorable exposure to radiation during treatment which may explain why some points of exposed human skin do develop into a skin condition whereas others does not. For example, in external RT there is an effect called a "buildup region": photons of the treatment beam TB impinge on the skin and cause a peak of dose just beneath the skin. Also, with external beams, tangential beams lead to more damage than beams entering at a steeper angle, such as orthogonally.

The longer the observation, that is, the more video feeds from previous sessions are included and concatenated with the current one and processed by the machine learning model M, the more accurate the prediction is likely going to be.

The output data as produced by the machine learning model M may be represented as a skin map *S*. A skin map *S* is a spatially resolved image structure so has image character, thus includes pixel values (*x,y*), but is in itself not an image as the frames Ft are, as the latter modulates with interrogator signal intensity, whilst, the earlier does not in general. Interrogator signal is the type of image causing signal used by the imager, such as IR, optical light, etc. If necessary (e.g. in lung or breast treatment without gating), the system may be configured to compensate for breathing motion so that a position $(x,y)$ corresponds to a given anatomical skin position.

In general, the size and the dimension of the skin map $S$ corresponds to that of the individual frames in the video feed. Each point $(x,y)$ of the map thus correspond to an on-skin position of the patient and each value at the respective position $(x,y)$ in the map is a numerically encoded indication for the likelihood of skin disease or condition to develop at the said position $(x,y)$. In other words, the machine learning model maps the time series of measurements $F_t(x,y)$ for a certain position $(x,y)$ on the skin as recorded in the stream $(F_t)$ to an indication of a likelihood or score for development of skin lesion (disease, injury, etc) at the respective on-skin position $(x,y)$. The indications in the map at each position $(x,y)$ may or may not correspond to probability. Preferably, scores are normalized in a bound range $[a,b]$, such as $a=0$ and $b=1$ for the unit interval but other ranges are also envisaged, as required and convenient. The skin map S may in addition or instead code for severity or type of injury.

The skin map S may be visualized by graphics display generator GGD in graphics display GDG. The graphics display GD may define a visualization surface configured so that grey or color/hue intensities are made to vary with score magnitude across the surface as per the skin map S. Akin to a "heat map", certain regions emerge in the surface that indicate skin portions estimated by model M to be at heightened risk of lesion development as compared to other skin regions. For different types of injury (chronic, acute, etc) or severity, respective different skin maps may be generated. A spatial surface map, extractable from a patient image for example, may be used to project the skin map S onto. This allows for improved spatial displaying so that user can quicker ascertain the results. Preferably, the patient image is the planning image. Preferably the patient image is 3D, such as a CT or MRI image. A spatial correspondence between the skin map $S$ as extracted by the sensors/computed by model M, and the surface model may be established. This can be done by image registration or by some pre-session calibration between the cameras and the patient.

Instead of, or in addition to, displaying the predicted output (eg,the skin map) as computed by the model M, this may be forwarded to the planning module PM. The plan may then be adjusted automatically or by the user considering the information in the skin map $S$ to create an adapted plan with targeted sparing (e.g. by redefining or adding dose constraints, delivery angles, etc). The skin map $S$ may be compared to the planned skin dose. In particular, as observed above, a plan that envisages more tangential beams is probably less desirable than one with fewer tangential beams. The plan may thus be changed to reduce tangential beam impact.

Components of the monitoring system SYS are now explained in more detail.

One or more camera systems may be used as sensors SN. The sensors SN may be mounted on a dedicated tripod or other holders, suitably aligned with the field of view to capture the exposed (not covered by cloth) skin portion of the patient during the treatment session. The sensors SN may be floor-, ceiling-, or wall-mounted in the treatment room where the radiation is delivered, as required. As said, more than one sensor spatially distributed may be so mounted. Preferably, installation is configured to reduce (or altogether avoid) field-of-view occlusion. Furthermore, the camera(s) preferably do not obstruct or otherwise mechanically interfere with the delivery apparatus during operation. The camera SN installation can be fixed or mobile. The camera(s) SN's FOV is preferably aligned to capture imagery for selected skin areas that are generally known to be prone to injury, e.g. the armpit for breast treatment. In embodiments, the camera(s) SN are mounted on the treatment device TD, such as on the treatment head of the linac. This ensures that the camera SN can image skin at any angle independent of treatment head position, at least for positions above the treatment table (on which patient my lie during RT). Other mounting/installation options for camera SN at the delivery device TD, or the treatment table are also envisaged.

Different types of video cameras are envisaged herein for such sensor(s) SN, which may include, in addition to the above-mentioned options, LIDAR, or other depth sensing camera technology. The latter can pick up even subtle changes to the skin surface geometry including the crests and trough patterns mentioned earlier. Depth sensing data may be used to establish a correspondence between the (x, y) coordinates of the video and the patient surface. In addition or instead, the depth sensing data may be used to calibrate the other sensors SN to the skin/patient surface data as may be extracted from CT volume of the patient. This information may be used to create the skin surface map mentioned above.

Any two or more of the above mentioned camera SN types may be used in combination so the frames may be represented and stored as multichannel data $F_t^k$, with index k indicating the channel, such as optical light intensity, depth profile of skin, prefusion level, temperature etc, and index $t$ indicating, as before, frame acquisition time $t$.

The synchronizer SYNC operates so that data recording preferably starts before the treatment beam is turned on. This allows for post-treatment calibration. Different options for data recording may be used such as permanent or gated. The gating signal intercepted by synchronizer SYNC may be the beam angle $\alpha$. Additional recording before and after RT beam-on/off events may be done to capture time-lagged onset of skin reaction.

Reference is now made to Fig. 3 which shows an implementation of the machine learning model M according to one embodiment.

The machine learning model M may be implemented as shown of the (artificial) neural network (NN) type. Overall structure of the NN may be one of a feed -forward neural network. Preferably however, the NN may be configured as a recurrent network to more robustly process the time series input, such as is the video feed $F_t$.

A convolutional network is preferably used, as such networks have proved to be particularly suited to process image type data such as is the case here.

The machine learning task mainly envisaged herein is one of regression, where the input video feed of suitable length is regressed into the skin map *S*. However, classifier type networks M are also envisaged, where for each position a category is estimated for the risk of skin lesion developments, such as "*LOW*", "*MEDIUM*" and "*HIGH*" (which is an example of 3 categories), but or more or less categories as also envisaged herein. A binary map may be computed for example with scores for two categories: *"NO/LOW RISK", "YES/HIGH RISK",* etc.

The input data may be suitably arranged in matrix or tensor type form. Individual frames across time may be arranged in "different channels" as "slices" of a tensor structure for example. More such channels may be added for the contextual data, the input data thus being formed as a higher dimensional tensor. The input data can so be consolidated in tensor or matrix structures can be efficiently be co-processed, especially by processors with parallel processing capabilities, such as GPUs or others. The video feed, and possibly the contextual data, stored in such a multidimensional structure may then be propagated as a whole through processing layers Lj of the network. The network M includes one, but preferably more than one, hidden layers Lj arranged in cascaded manner between input layer IL and output layer OL.

Convolutional operators CV may operate in-channel or across channels to produce intermediate output data at a given layer $L_j$. The intermediate output data is passed through nonlinear operators to produce feature maps for that layer $L_j$. The feature maps are then passed on to the next layer to obtain in the same manner higher generation feature maps and so forth in a cascaded manner. The nonlinear operators may include rectified liner units (RELU as exemplary shown in Fig. 3), sigmoid-functions, *tanh*-functions, etc. Additional operators per layer of other type may be used, such as pooling layers PL, drop-out layers (not shown), etc.

The final layer OL is a regression or classification layer that may be implemented in one or more fully connected layers as opposed to the convolutional layers that are not fully connected in the previous hidden layers. For classification, the output layer may be configured as a *softmax-layer.* In regression. the output layer OL is formed by one more fully connected, (linear) layer, as opposed to the non-fully connected hidden ("deep") layers Lj upstream. Other models, not necessarily of the NN type may be used instead, such as SVM, random forest, decision trees, statistical regression/classification techniques, etc. Thus, the NN model M in Figure is merely one example embodiment.

The predictor logic PL is preferably configured for real time monitoring. The skin map S is output and adapted during the recording in the treatment session. The graphics display generator may cooperate with the model M to dynamically update the skin map visualization as the frames in the stream are processed by the logic PL's model M. Quick turnaround may be desirable for better real-time experience, so as to be able to timely take remedial action for skin protection /treatment. The model M may thus be preferably implemented on computer system(s) with high performance processor(s), of general or of dedicated type. Multicore processor(s) may be used to take advantage of parallelized processing. GPUs (graphical processing units) may preferably be used for deployment and/or training, in particular when matrix/tensor structures are used to represent the input data as mentioned above.

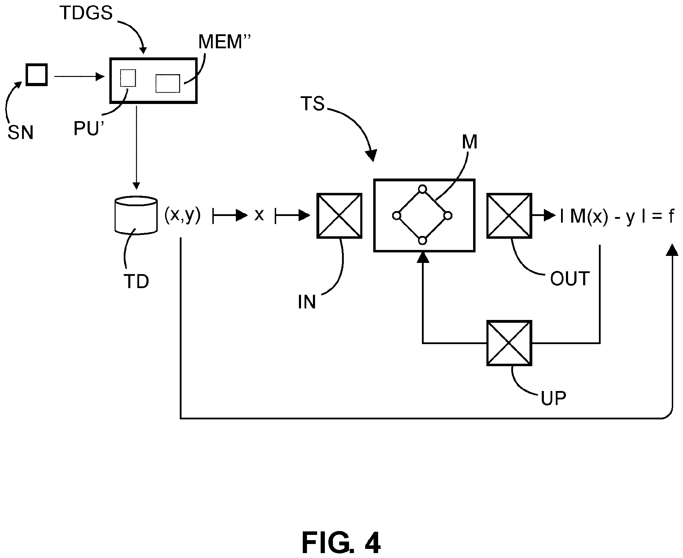

Reference is now made to Fig. 4 which shows a training system TS that may be used to train the machine learning model M based on training data. The training data may be procured from medical databases that includes data from different patients from historic (previous) radiation treatment sessions. Instead of or on addition, training data may be generated synthetically. Data augmentation techniques may be used to induce greater variation in the training data to boost learning.

It is preferably supervised learning that is envisaged herein where the training data retrievable from the medical database(s) is suitably labelled to define instance $(x,y)^i$ of training input data $(x^i)$ and their associated target $(y^i)$. In this case, the target corresponds to indications of localized skin conditions that may have developed in the historic patient case.

The training input data *x* represents historic video footage of skin acquired during training sessions, preferably using the same camera types and setup as described above for Fig. 2. Such footage may not necessarily be readily available, so could be generated in a training data collection phase at one or more clinical sites over course of weeks or months using the camera setup as described above in Fig. 2. The cameras SN are pre-installed before the model training phase. Thus, the cameras in the collection phase prior to training phase form a training data generator system TDGS to collect the training data inputs *x*. The labelling for training purposes may be done by a human expert in one or more labelling sessions in which the collected footage is reviewed for skin injury, severity, lesion type etc, and documented in suitable annotations to obtain associated targets *y*. The training data generator system TDGS may further comprise memory MEM" to store the collected data and a processor unit PU' to process the data. For example, the processor unit PU' may receive instructions from, for example, a user (medical expert) operable annotation tool (not shown) to record associated targets *y*. The training inputs x and their targets *y* may be stored in association to obtain training data.

In more detail, and in some embodiments, certain skin maps representative of portions of the human skin that were exposed during the respective historic treatment sessions may be prepared to obtain targets *y*. For this, future historic health records of patients that underwent radiation treatment sessions may need to be monitored and analyzed repeatedly at times for a certain period to detect evidence (eg textual entries in health records) for the development of skin cancer or other skin conditions. Such evidence may be localized by a text search tool for example. A human annotator annotates in the training map for that patient the skin condition at the correct location. A scripting tool may be used to automatically query database for new entries that fit a predefined pattern including suitable keywords, such as *"skin", derma*"*. Wildcard constructs ("*") may be used. Scheduled searches using a script programmed in a database search language may be set up. The script may be repeatedly executed at a predefined schedules (weeks, monthly, etc). The described database query may be run as part of the above-mentioned training data collection phase. The expert annotation may not necessarily be done on a pixel-by-pixel basis, but may be done instead per regions (sub-sets of pixels) to roughly indicate where the skin condition has developed. Care should be taken that the skin conditions found in the historic records can be reasonably said to have originated from the previous treatment sessions. Dermatological domain knowledge may need to be consulted for this safeguard. Expert annotation may include annotating not only for location and/or severity, but also for type of injury, or, instead of location, annotation is for type of injury.

Training data may be derived from PROMs, or regular clinical surveillance by annotating a patient surface model accordingly using type and severity of the injury.

As another option for sourcing training target data, data indicative of type and/or severity of skin injury is extracted automatically from camera data, based on a comparison between pre-treatment skin status on the day of the first/earlier fraction to the corresponding pre-treatment skin status on one or more later fractions. Late effects may be captured by re-imaging the respective body regions with the same set of cameras during post-treatment surveillance, for example as part of the training data generator system TDGS. This type of camera-based surveillance may be used during the above-mentioned training data collection phase.

With continued reference to Fig. 4 and turning now in more detail to the training phase, this includes a process of adapting parameters of the model based on the training data. An explicit model is not necessarily required as in some examples it is the training data itself that constitutes the model M, such as in clustering techniques or k-nearest neighbors. etc. In explicit modelling, such as in NN-based approaches and many others, the model may include as system of model functions/computational nodes, with their input and/or output at least partially interconnected. The model functions or nodes are associated with parameters θ which are adapted in training. The model functions may include the convolution operators and/or weights of the non-linear units such as a RELU, mentioned above at Fig. 3 in connection with NN-type models. In the NN-case, the parameters θ may include the weights of convolution kernels of operators CV and/or of the non-linear units. The parameterized model may be formally written as $M^\theta$. The parameter adaptation may be implemented by a numerical optimization procedure. The optimization may be iterative. An objective function $f$ may be used to guide or control the optimization procedure. The parameters are adapted or updated by an updater UP so that the objective function is improved. The input training data $x^i$ is applied to the model. The model responds to produce training data output $M(x^i) = \tilde{y}^i$. The objective function is a mapping from parameters space into a set of numbers. The objective function $f$ measures a combined deviation between the training data outputs $\tilde{y}^i$ and the respective targets $y^i$. Parameters θ are iteratively adjusted to that the combined deviation decreases until a user or designer pre-set stopping condition is satisfied. The objective function may use a distance measure $\| . \|$ to quantify the deviation.

In some embodiments, but not all, the combined deviation may be implemented as a sum over some or all residues based on the training data instances/pairs $(x^i, y^i)^i$, and the optimization problem in terms of the objective function may be formulated as:

$$argmin_\theta f = \sum_k \| M^\theta(x_k), y_k \| \qquad (1)$$

In the setup (1), the optimization is formulated as a minimization of cost function $f$, but is not limiting herein, as the dual formulation of maximizing a utility function may be used instead. Summation is over training data instances $k$.

The cost function $f$ may be pixel/voxel-based, such as the L1 or L2-norm cost function. For example in least squares or similar approaches, the (squared) Euclidean-distance-type cost function in (1) may be used for the above mentioned regression task when comparing training output skin maps vs their target skin maps. When configuring the model as a classifier, the summation in (1) is formulated instead as one of cross-entropy or Kullback-Leiber divergence or similar.

The exact functional composition of the updater UP depends on the optimization procedure implemented. For example, an optimization scheme such as backward/forward propagation or other gradient based methods may then be used to adapt the parameters $\theta$ of the model M so as to decrease the combined residue for all or a subset $(x_k, y_k)$ of training pairs from the full training data set. Such subsets are sometime referred to as batches, and the optimization may proceed batchwise until all of the training data set is exhausted, or until a predefined number of training data instances have been processed. Batch-normalization may be used to avoid adverse numerical effects. Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

Training may be a one-off operation, or may be repeated once new training data become available. The training system as shown in Fig. 4 can be considered for all learning schemes, in particular supervised schemes. Unsupervised learning schemes may also be envisaged herein in alternative embodiments. GPUs may be used to implement the training system TS.

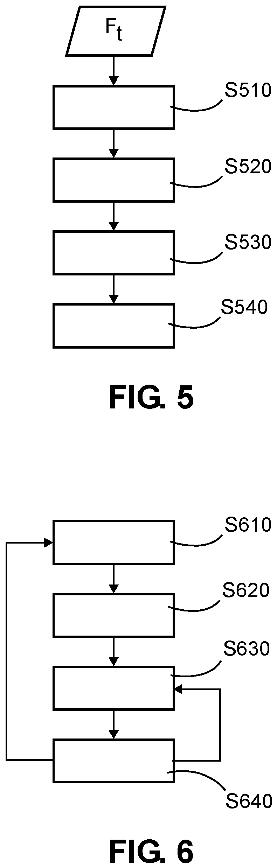

Reference is now made to Fig. 5 which shows a flow chart of a method of supporting radiation treatment/therapy. More particularly, the computer-implemented method includes steps to obtain a spatially resolved skin map of a patient with indications of skin conditions or injuries/severity thereof developing at a given on-skin position (pixel or regions) as a result of prior exposure to radiation during radiation therapy.

At step S510 input data is received. The input may include in particular a video feed of imagery acquired during one or more imaging sessions of region(s) of patient's skin. In particular, the input data may further comprise contextual data such as treatment parameters and/or control parameters used. Such treatment/control parameters are sometimes stored in log files and can be easily obtained.

At step S520, the input data, in particular the video feed, is applied to a trained machine learning model, previously trained on training data as described herein. The training machine learning model processes the input data to produce the skin map $S$.

At step S530 the skin map is made available such as by storage and/or by being displayed on a display device, or other.

The skin map may be superimposed on the planning image or other image of the patient suitably registered, if required, to provide a spatial result indication of where on the patient's exposed skin, skin injuries may develop. Entries of the skin map represent prediction scores, such as probability, for a skin's condition to develop at a given on-skin position/region, caused by the radiation exposure.

At step S540, the skin map may be used to re-plan the current plan. In particular, if certain portions of the patient's skin are identified as possible locations where a skin condition may develop, the patient may be re-positioned and the plan re-calculated accordingly so as to avoid or reduce exposure of those at-risk skin locations. In particular, the above described planning algorithm may need to be re-run, taking account the newly required spatial position of the patient to better avoid/reduce at-risk skin exposure.

In particular, a new plan may be computed where the skin portions are avoided which may necessitate further and/or different dosage constraints to those used in the original plan.

Replanning step S540 is however optional. It may be sufficient and more efficient in some case to cover the at-risk skin portions with protective material. In addition or instead, the fractions may be rescheduled to insert additional recovery (non-treatment) intervals, such as more days, etc. Reference is now made to Fig. 6 which shows steps of training method based on an iterative optimization procedure.

At step S610, training data is received in the form of pairs/instances $(x^k, y^k)$. Each pair includes the training input $x_k$ and the associated target $y_k$. $x_k$, as defined above.

At step S620, the training input $x_k$ is applied to an initialized machine learning model M to produce a training output. Initialization may include assigning random and/or constant parameters to the model.

A deviation, or residue, of the training output $M(x_k)$ from the associated target $y_k$ is quantified S630 by a cost function $f$. One or more parameters of the model are adapted at step S640 in one or more iterations in an inner loop to improve the cost function. For instance, the model parameters are adapted to decrease residues as measured by the cost function.

The training method then returns in an outer loop to step S610 where the next pair of training data is fed in. In step S620, the parameters of the model are adapted so that the aggregated residues of all pairs considered are decreased, in particular minimized. The cost function quantifies the aggregated residues. Forward- backward propagation or similar gradient-based techniques may be used in the inner loop.

More generally, the parameters of the model M are adjusted to improve objective function $f$ which is either a cost function or a utility function. In embodiments, the cost function is configured to measure the aggregated residues. In embodiments the aggregation of residues is implemented by summation over all or some residues for all pairs considered. In particular, in preferred embodiments the outer summation proceeds in batches (subset of training instances), whose summed residues are considered all at once when adjusting the parameters in the inner loop. the outer loop then proceeds to the next batch and so for until the requisite number of training data instances have been processed.

The method may include a preparatory step S605, where the training is generated or procured. This step may implement a training data collection phase as mentioned above, where sensors SN such as cameras are installed in treatment rooms, to monitor skin for a number of patients over time during treatment. In addition, pre- and post- treatment imagery may be collected. The imagery may be reviewed and annotated to obtain the skin map targets $y^k$. The video footage during treatment then forms the training input data $x^k$, for a number of patients $i = 1...N$.

In general, task performance by the machine learning model may improve measurably, with training experience. Training experience includes the said training data and exposure of the model to such data. Task performance may improve the better the data represents the task to be learned and the generally the more data is used: *"Training experience helps improve performance if the training data well represents a distribution of examples over which the final system performance is measured"*. See T. M. Mitchell, "Machine Learning", page 2, section 1.1, page 6, 1.2.1, McGraw-Hill, 1997. The performance may be measured by objective tests based on output produced by the module in response to feeding the model with test data. The performance may be defined in terms of a certain error rate to be achieved for the given test data.

The method may be implemented on one or more general-purpose processing units TS, preferably having processors capable for parallel processing to speed up the training.

The components of the training system TS may be implemented as one or more software modules, run on one or more general-purpose processing units PU such as a workstation associated with the therapy system TPS, or on a server computer associated with a group of such systems.

The proposed monitoring system SYS and method may be used, instead of in radiation therapy, in imaging sessions where ionizing radiation is used, such as in X-ray imaging. Applications outside the clinical realm are also envisaged, such as during sun-bathing, either outside or in sunbeds or tanning booths. In commercial sunbed studios, the skin reaction sensors SM may be installed in the sunbed/booths. For outside use by the recreational user, such as when out at the beach or by-the-pool, the system logic may be useable as a user-installable program ("app") on the user's end device, such as a tablet computer or smartphone. The processing may be done wholly or partly on a server to which user connects, or may be done wholly or partly locally on the user's end device. Some user end devices already include AI/ML-engines or are equipped with multi-core processors that allow securing fast return time. The native camera equipment of current tablets or smartphones may be used as the above-described skin monitoring sensors SN. The user may need to ensure by the camera's FoV is correctly aligned with the exposed skin portion. A tripod or similar positioning/holder devices may be uses to aid in correct alignment.

In this case of the above-mentioned leisure application, the training data or contextual information may be supplied during use in data collection phase. User may calibrate / train the system for themselves, by providing feedback about the (late) effects of a sunbath so that the prediction becomes more personalized. Video footage is acquired over a certain time period of a certain skin potion of concern, depending in camera FOV during data collection phase. The feedback is associated with the footage to obtain training data. The feedback may include a training skin map, with annotations provided by user, suitably encoded to indicate skin location and type of injury /severity thereof, etc.

With the proposed system, user may indulge in sunbathing in a skin-safe manner, eg, by taking a video of their skin before, during and after sunbathing and receive a quality measure of tan vs injury for example.

The components of system SYS may be implemented as one or more software modules, run on one or more general-purpose processing units PU such as a workstation associated with the imager or treatment device TD or on a server computer associated with a group of imagers.

Alternatively, some or all components of the system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, the system SYS may be implemented in both, partly in software and partly in hardware.

The different components of the system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A system (SYS) for monitoring exposure to radiation, comprising:

   input interface (IN) or receiving input data including sensed data of a portion of a subject's skin acquired by at least one sensor over time,
   A predictor logic (PL) configured to process the said input data to compute output data including a map (S) representative of spatially resolved magnitudes and/or type of predicted skin reactions to radiation exposure; and an output interface (OUT) to output said map.

2. A system of claim 1, comprising a graphics display generator (GDG) configured to cause displaying, on a display device (DD), a graphics display of the map.

3. System of any one of the previous claims, wherein the one or more sensors include any one or more of: i) optical camera, ii) depth or range sensing camera, iii) IR or near-IF camera.

4. System of any of the previous claims, wherein the input data further includes one or more control parameter(s) for a radiation treatment delivery apparatus and/or treatment delivery parameter(s).

5. System of any of the previous claims, wherein the input data includes vital sign measurements in relation to the subject, including any one or more of i) skin temperature, ii) oxygenation, iii) pule rate, iv) blood pressure.

6. System of any one of the previous claims, wherein the predictor logic (PL) is based on a machine learning model (M).

7. System of any one of the previous claims, wherein the sensed data is acquired during radiation exposure.

**8.** System of any one the previous claims, wherein the said radiation exposure is one during any one of: i) radiation therapy, ii) X-ray imaging, iii) sunbathing, iv) UV-sunbed use.

**9.** A system (TS) for training the machine learning model of claim 6, based on training data.

**10.** System (SN, TDGS) for procuring or generating training data for use in training the machine learning model of claim 6, based the training data.

**11.** A computer-implemented method of monitoring exposure to radiation, comprising:

receiving (S510) input data including sensed data of a portion of a subject's skin acquired by at least one sensor over time,
processing (S520) the said input data to compute output data including a map representative of spatially resolved magnitudes and/or type of predicted skin reactions to radiation exposure; and
outputting (S530) said map.

**12.** A method for training the machine learning model of claim 6, based on training data.

**13.** A method for procuring or generating training data for use in training the machine learning model of claim 6, based on training data.

**14.** A computer program element, which, when being executed by at least one processing unit, is adapted to cause the processing unit to perform the method as per any one of claims 11-13.

**14.** At least one computer readable medium having stored thereon the program element of claim 14, or having stored thereon the machine learning model as per claim 6.

**Amended claims in accordance with Rule 137(2) EPC.**

**1.** A system (SYS) for monitoring exposure to radiation, comprising:

input interface (IN) or receiving input data including sensed data of a portion of a subject's skin acquired by at least one sensor over time,
A predictor logic (PL) configured to process the said input data to compute output data including a map (5) representative of spatially resolved magnitudes and/or type of predicted skin reactions to radiation exposure; and
an output interface (OUT) to output said map.

**2.** A system of claim 1, comprising a graphics display generator (GDG) configured to cause displaying, on a display device (DD), a graphics display of the map.

**3.** System of any one of the previous claims, wherein the one or more sensors include any one or more of: i) optical camera, ii) depth or range sensing camera, iii) IR or near-IF camera.

**4.** System of any of the previous claims, wherein the input data further includes one or more control parameter(s) for a radiation treatment delivery apparatus and/or treatment delivery parameter(s).

**5.** System of any of the previous claims, wherein the input data includes vital sign measurements in relation to the subject, including any one or more of i) skin temperature, ii) oxygenation, iii) pule rate, iv) blood pressure.

**6.** System of any one of the previous claims, wherein the predictor logic (PL) is based on a machine learning model (M).

**7.** System of any one of the previous claims, wherein the sensed data is acquired during radiation exposure.

**8.** System of any one the previous claims, wherein the said radiation exposure is one during any one of: i) radiation therapy, ii) X-ray imaging, iii) sunbathing, iv) UV-sunbed use.

**9.** A system (TS) for training the machine learning model of claim 6, based on training data.

10. System (SN, TDGS) for procuring or generating training data for use in training the machine learning model of claim 6, based the training data.

11. A computer-implemented method of monitoring exposure to radiation, comprising:

    receiving (S510) input data including sensed data of a portion of a subject's skin acquired by at least one sensor over time,
    processing (S520) the said input data to compute output data including a map representative of spatially resolved magnitudes and/or type of predicted skin reactions to radiation exposure; and
    outputting (S530) said map.

12. A method for training the machine learning model of claim 6, based on training data.

13. A method for procuring or generating training data for use in training the machine learning model of claim 6, based on training data.

14. A computer program element, which, when being executed by at least one processing unit, is adapted to cause the processing unit to perform the method as per any one of claims 11-13.

15. At least one computer readable medium having stored thereon the program element of claim 14, or having stored thereon the machine learning model as per claim 6.

**FIG. 1**

**FIG. 2**

**FIG. 3**

**FIG. 4**

$F_t$

S510

S520

S530

S540

# FIG. 5

S610

S620

S630

S640

# FIG. 6

Europäisches
Patentamt

European
Patent Office

Office européen
des brevets

## EUROPEAN SEARCH REPORT

**Application Number**

EP 21 19 7632

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/136455 A1 (BHARAT SHYAM [US] ET AL) 19 May 2016 (2016-05-19) | 1-5,7,8, 11,14 | INV. A61N5/10 |
| Y | * paragraph [0002] * | 6,9,10, | A61B5/00 |
|   | * paragraph [0004] * | 12,13,15 | A61B5/01 |
|   | * paragraph [0010] * | | A61B6/00 |
|   | * paragraph [0014] * | | G06T7/00 |
|   | * paragraph [0021] * | | |
|   | * paragraph [0022] * | | |
|   | * paragraph [0023] * | | |
|   | * paragraph [0024] * | | |
|   | * paragraph [0027] * | | |
|   | * paragraph [0030] * | | |
|   | * paragraph [0031] * | | |
|   | * paragraph [0033] * | | |
|   | * paragraph [0036] * | | |
|   | * paragraph [0037] * | | |
|   | * paragraph [0042] * | | |
|   | * paragraph [0043] * | | |
|   | * paragraph [0044] * | | |
|   | * paragraph [0005] * | | |
|   | * paragraph [0034] * | | |
|   | ————— | | |
|   | -/-- | | TECHNICAL FIELDS SEARCHED (IPC) |
|   | | | A61N |
|   | | | A61B |
|   | | | G06T |

The present search report has been drawn up for all claims

| Place of search | Date of completion of the search | Examiner |
|---|---|---|
| Munich | 1 March 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

**EUROPEAN SEARCH REPORT**

Application Number

EP 21 19 7632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2017/128748 A1 (MATUBARA HIROAKI [JP] ET AL) 11 May 2017 (2017-05-11)<br>* paragraph [0001] *<br>* paragraph [0008] *<br>* paragraph [0009] *<br>* paragraph [0017] *<br>* paragraph [0023] *<br>* paragraph [0032] *<br>* paragraph [0041] *<br>* paragraph [0042] *<br>* paragraph [0071] *<br>* paragraph [0072] *<br>* paragraph [0073] *<br>* paragraph [0104] *<br>* paragraph [0112] *<br>* paragraph [0115] *<br>* paragraph [0116] *<br>* paragraph [0118] *<br>* paragraph [0124] *<br>* paragraph [0135] *<br>----- | 1-5,7,8,<br>11<br>6,9,10,<br>12,13,15 | |
| Y | US 2014/321728 A1 (CHIN MICHAEL S [US]) 30 October 2014 (2014-10-30)<br>* paragraph [0003] *<br>* paragraph [0005] *<br>* paragraph [0013] *<br>* paragraph [0014] *<br>* paragraph [0029] *<br>* paragraph [0030] *<br>* paragraph [0033] *<br>* paragraph [0044] *<br>* paragraph [0046] *<br>* paragraph [0052] *<br>* paragraph [0054] *<br>* paragraph [0055] - paragraph [0056] *<br>* paragraph [0060] *<br>-----<br>-/-- | 1-15 | TECHNICAL FIELDS SEARCHED (IPC) |

The present search report has been drawn up for all claims

| Place of search | Date of completion of the search | Examiner |
|---|---|---|
| Munich | 1 March 2022 | Rodríguez Cosío, J |

EPO FORM 1503 03.82 (P04C01)

Europäisches
Patentamt

European
Patent Office

Office européen
des brevets

**EUROPEAN SEARCH REPORT**

Application Number

EP 21 19 7632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/193597 A1 (FAN WENSHENG [US] ET AL) 18 June 2020 (2020-06-18)<br>* paragraph [0007] *<br>* paragraph [0010] *<br>* paragraph [0049] *<br>* paragraph [0059] *<br>* paragraph [0133] *<br>* paragraph [0144] *<br>* paragraph [0146] *<br>* paragraph [0153] *<br>* paragraph [0161] *<br>----- | 6,9,10,<br>12,13,15 | |
| Y | US 2017/115162 A1 (DUMONT EMMANUEL [US] ET AL) 27 April 2017 (2017-04-27)<br>* paragraph [0006] *<br>* paragraph [0009] *<br>* paragraph [0010] *<br>* paragraph [0118] *<br>* paragraph [0119] *<br>----- | 6,9,10,<br>12,13,15 | |
| Y | US 2019/030359 A1 (DIJKSTRA ALAIN [NL] ET AL) 31 January 2019 (2019-01-31)<br>* paragraph [0002] *<br>* paragraph [0018] *<br>* paragraph [0065] *<br>* paragraph [0068] *<br>* paragraph [0071] *<br>* paragraph [0079] *<br>* claim 4 *<br>----- | 6,9,10,<br>12,13,15 | **TECHNICAL FIELDS SEARCHED (IPC)** |
| Y | WO 2017/074505 A1 (SPECTRAL MD INC [US]) 4 May 2017 (2017-05-04)<br>* paragraph [0006] *<br>* paragraph [0312] *<br>* paragraph [0348] *<br>* paragraph [0393] *<br>* paragraph [0416] *<br>----- | 1-15 | |

The present search report has been drawn up for all claims

| Place of search | Date of completion of the search | Examiner |
|---|---|---|
| Munich | 1 March 2022 | Rodríguez Cosío, J |

EPO FORM 1503 03.82 (P04C01)

## EP 4 151 276 A1

## ANNEX TO THE EUROPEAN SEARCH REPORT
## ON EUROPEAN PATENT APPLICATION NO.

EP 21 19 7632

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2022

| Patent document cited in search report | | Publication date | Patent family member(s) | | Publication date |
|---|---|---|---|---|---|
| US 2016136455 | A1 | 19-05-2016 | CN | 105358218 A | 24-02-2016 |
| | | | EP | 3013419 A1 | 04-05-2016 |
| | | | JP | 6259078 B2 | 10-01-2018 |
| | | | JP | 2016529953 A | 29-09-2016 |
| | | | US | 2016136455 A1 | 19-05-2016 |
| | | | WO | 2014207663 A1 | 31-12-2014 |
| US 2017128748 | A1 | 11-05-2017 | EP | 3124077 A1 | 01-02-2017 |
| | | | JP | 6448142 B2 | 09-01-2019 |
| | | | JP | WO2015146164 A1 | 13-04-2017 |
| | | | US | 2017128748 A1 | 11-05-2017 |
| | | | WO | 2015146164 A1 | 01-10-2015 |
| US 2014321728 | A1 | 30-10-2014 | CA | 2856538 A1 | 06-06-2013 |
| | | | EP | 2785247 A1 | 08-10-2014 |
| | | | US | 2014321728 A1 | 30-10-2014 |
| | | | WO | 2013082192 A1 | 06-06-2013 |
| US 2020193597 | A1 | 18-06-2020 | US | 2020193597 A1 | 18-06-2020 |
| | | | US | 2021201479 A1 | 01-07-2021 |
| US 2017115162 | A1 | 27-04-2017 | US | 2017115162 A1 | 27-04-2017 |
| | | | US | 2017118854 A1 | 27-04-2017 |
| | | | US | 2019145820 A1 | 16-05-2019 |
| | | | WO | 2017035384 A1 | 02-03-2017 |
| US 2019030359 | A1 | 31-01-2019 | EP | 3632507 A1 | 08-04-2020 |
| | | | US | 2019030359 A1 | 31-01-2019 |
| WO 2017074505 | A1 | 04-05-2017 | CN | 108471949 A | 31-08-2018 |
| | | | EP | 3367887 A1 | 05-09-2018 |
| | | | JP | 6785307 B2 | 18-11-2020 |
| | | | JP | 2019502418 A | 31-01-2019 |
| | | | KR | 20180078272 A | 09-07-2018 |
| | | | WO | 2017074505 A1 | 04-05-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A camera-based multispectral setup for remote vital signs assessment. **M PAUL et al.** EMBEC & NBC 2017, IFMBE Proceedings. Springer, 2018, vol. 65, 968-971

- **T. M. MITCHELL.** Machine Learning. McGraw-Hill, 1997